(19) Europäisches Patentamt
European Patent Office
Office européen des brevets

(11) **EP 4 024 170 B1**

(12) **FASCICULE DE BREVET EUROPEEN**

(45) Date de publication et mention de la délivrance du brevet:
**25.10.2023  Bulletin 2023/43**

(51) Classification Internationale des Brevets (IPC):
**G06F 3/01** *(2006.01)*   **A61B 5/00** *(2006.01)*
**A61B 5/16** *(2006.01)*   **A61B 5/37** *(2021.01)*

(21) Numéro de dépôt: **21218205.9**

(22) Date de dépôt: **29.12.2021**

(52) Classification Coopérative des Brevets (CPC):
**G06F 3/015; A61B 5/165; A61B 5/37;**
**A61B 5/7264; A61B 5/7267;** G06F 2203/011

(54) **MÉTHODE D'APPRENTISSAGE AUTO-ADAPTATIF D'UNE INTERFACE NEURONALE DIRECTE UTILISANT UNE DÉTECTION PHYSIQUE D'ÉTAT MENTAL**

SELBSTLERNMETHODE EINER DIREKTEN NEURONALEN SCHNITTSTELLE UNTER VERWENDUNG EINER PHYSISCHEN ERFASSUNG DES MENTALEN ZUSTANDS

METHOD FOR SELF-ADAPTIVE LEARNING OF A DIRECT NEURAL INTERFACE USING A PHYSICAL DETECTION OF MENTAL STATE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.12.2020  FR 2014199**

(43) Date de publication de la demande:
**06.07.2022  Bulletin 2022/27**

(73) Titulaire: **Commissariat à l'énergie atomique et aux énergies alternatives**
**75015 Paris (FR)**

(72) Inventeurs:
• **ROUANNE, Vincent**
**38054 GRENOBLE CEDEX 09 (FR)**
• **AKSENOVA, Tetiana**
**38054 GRENOBLE CEDEX 09 (FR)**

(74) Mandataire: **Brevalex**
**56, Boulevard de l'Embouchure**
**B.P. 27519**
**31075 Toulouse Cedex 2 (FR)**

(56) Documents cités:
• **DAVID ROTERMUND ET AL: "Towards On-line Adaptation of Neuro-prostheses with Neuronal Evaluation Signals", BIOLOGICAL CYBERNETICS ; ADVANCES IN COMPUTATIONAL NEUROSCIENCE, SPRINGER, BERLIN, DE, vol. 95, no. 3, 27 juin 2006 (2006-06-27), pages 243-257, XP019427250, ISSN: 1432-0770, DOI: 10.1007/S00422-006-0083-7**
• **GÜREL TAYFUN ET AL: "Unsupervised adaptation of brain-machine interface decoders", FRONTIERS IN NEUROSCIENCE, 16 novembre 2012 (2012-11-16), XP055841383, DOI: 10.3389/fnins.2012.00164**
• **TOMISLAV MILEKOVIC ET AL: "Paper;Error-related electrocorticographic activity in humans during continuous movements;Error-related electrocorticographic activity in humans during continuous movements", JOURNAL OF NEURAL ENGINEERING, INSTITUTE OF PHYSICS PUBLISHING, BRISTOL, GB, vol. 9, no. 2, 13 février 2012 (2012-02-13), page 26007, XP020221605, ISSN: 1741-2552, DOI: 10.1088/1741-2560/9/2/026007**

EP 4 024 170 B1

- ITURRATE IÑAKI ET AL: "Teaching brain-machine interfaces as an alternative paradigm to neuroprosthetics control", SCIENTIFIC REPORTS , vol. 5, no. 1 10 septembre 2015 (2015-09-10), XP055840707, DOI: 10.1038/srep13893 Extrait de l'Internet: URL:https://europepmc.org/backend/ptpmcren der.fcgi?accid=PMC4564803&blobtype=pdf [extrait le 2021-09-13]
- BHATTACHARYYA SAUGAT ET AL: "Motor imagery and error related potential induced position control of a robotic arm", IEEE/CAA JOURNAL OF AUTOMATICA SINICA, vol. 4, no. 4, 15 septembre 2017 (2017-09-15), pages 639-650, XP011660555, ISSN: 2329-9266, DOI: 10.1109/JAS.2017.7510616 [extrait le 2017-09-15]
- DOMINIK WELKE ET AL: "Brain Responses During Robot-Error Observation", ARXIV.ORG, CORNELL UNIVERSITY LIBRARY, 201 OLIN LIBRARY CORNELL UNIVERSITY ITHACA, NY 14853, 4 août 2017 (2017-08-04), XP081290368, DOI: 10.17185/DUEPUBLICO/44533
- SALAZAR-GOMEZ ANDRES F ET AL: "Correcting robot mistakes in real time using EEG signals", 2017 IEEE INTERNATIONAL CONFERENCE ON ROBOTICS AND AUTOMATION (ICRA), IEEE, 29 mai 2017 (2017-05-29), pages 6570-6577, XP033127519, DOI: 10.1109/ICRA.2017.7989777
- RAKSHIT ARNAB ET AL: "Robotic link position control using brain computer interface", 2016 INTERNATIONAL CONFERENCE ON MICROELECTRONICS, COMPUTING AND COMMUNICATIONS (MICROCOM), IEEE, 23 janvier 2016 (2016-01-23), pages 1-6, XP032931001, DOI: 10.1109/MICROCOM.2016.7522567
- KUMAR AKSHAY ET AL: "A Review of Error-Related Potential-Based Brain-Computer Interfaces for Motor Impaired People", IEEE ACCESS, vol. 7, 26 septembre 2019 (2019-09-26), pages 142451-142466, XP011750268, DOI: 10.1109/ACCESS.2019.2944067

**Description**

**DOMAINE TECHNIQUE**

La présente invention concerne le domaine des interfaces neuronales directes encore dénommées BCI (*Brain Computer Interface*) ou BMI (*Brain Machine Interface*). Elle trouve notamment à s'appliquer à la commande neuronale directe d'une machine, telle qu'un exosquelette ou un ordinateur.

**ÉTAT DE LA TECHNIQUE ANTÉRIEURE**

Les interfaces neuronales directes utilisent les signaux électro-physiologiques émis par le cortex cérébral pour élaborer un signal de commande. Ces interfaces neuronales ont fait l'objet de nombreuses recherches notamment dans le but de restaurer une fonction motrice à un sujet paraplégique ou tétraplégique à l'aide d'une prothèse ou d'une orthèse motorisée.

Les interfaces neuronales peuvent être de nature invasive ou non invasive. Les interfaces neuronales invasives utilisent des électrodes intracorticales (c'est-à-dire implantées dans le cortex) ou des électrodes corticales (disposées à la surface du cortex) recueillant dans ce dernier cas des signaux électrocorticographiques (ECoG). Les interfaces neuronales non invasives utilisent des électrodes placées sur le cuir chevelu pour recueillir des signaux électroencéphalographiques (EEG). D'autres types de capteurs ont été également envisagés comme des capteurs magnétiques mesurant les champs magnétiques induits par l'activité électrique des neurones du cerveau. On parle alors de signaux magnétoencéphalographiques (MEG).

Les interfaces neuronales directes utilisent avantageusement des signaux de type ECoG, présentant l'avantage d'un bon compromis entre biocompatibilité (matrice d'électrodes implantées à la surface du cortex) et qualité des signaux recueillis.

Les signaux ECoG ainsi mesurés doivent être traités afin d'estimer la trajectoire du mouvement désiré par le sujet et en déduire les signaux de commande de l'ordinateur ou de la machine. Par exemple, lorsqu'il s'agit de commander un exosquelette, l'interface BCI estime la trajectoire du mouvement désiré à partir des signaux électro-physiologiques mesurés et en déduit les signaux de contrôle permettant à l'exosquelette de reproduire la trajectoire en question. De manière similaire, lorsqu'il s'agit de commander un ordinateur, l'interface BCI estime par exemple la trajectoire souhaitée d'un pointeur ou d'un curseur à partir des signaux électro-physiologiques et en déduit les signaux de commande du curseur/pointeur.

L'estimation de trajectoire, et plus précisément celle des paramètres cinématiques (position, vitesse, accélération), est encore dénommée décodage neuronal dans la littérature. Le décodage neuronal permet notamment de commander un mouvement (d'une prothèse ou d'un curseur) à partir de signaux ECoG.

L'estimation de trajectoire et le calcul des signaux de contrôle de l'exosquelette ou de l'effecteur nécessite généralement une phase d'apprentissage ou de calibration préalable, dite *off-line*. Lors de cette phase, le sujet imagine, observe ou réalise un mouvement selon une trajectoire déterminée pendant un intervalle de calibration donné. Les signaux électrophysiologiques mesurés pendant cet intervalle sont exploités en relation avec cette trajectoire pour construire un modèle prédictif et plus précisément pour calculer les paramètres de ce modèle.

La validité du modèle prédictif est cependant limitée dans le temps en raison de la non-stationnarité des signaux neuronaux. Pour cette raison, il est nécessaire de procéder à une calibration en ligne (*on-line*) du modèle prédictif, c'est-à-dire au fur et à mesure que les signaux neuronaux sont observés et la commande appliquée.

L'article de D. Rotermund et al. intitulé "Towards On-line Adaptation of Neuro-prostheses with Neuronal Evaluation Signais" publié dans Biological Cybernetics - Advances In Computational Neuroscience, vol. 95, no. 3, juin 2006, DOI: 10.1007/S00422-006-0083-7 décrit le contrôle d'un bras de robot virtuel en utilisant des signaux neuronaux et un modèle prédictif, dans lequel un signal d'erreur qui représente la déviation d'une trajectoire désirée est utilisé pour déclencher une mise à jour des paramètres du modèle prédictif.

L'article de T. Gürel et al. intitulé "Unsupervised adaptation of brain-machine interface decoders" publié dans Frontiers in Neuroscience, novembre 2012, DOI: 10.3389/fnins.2012.00164 présente un aperçu des techniques d'adaptation non supervisée utilisables pour le décodage de signaux dans des interfaces neuronales directes pour éviter les dégradations de performances causées par la non-stationnarité des signaux neuronaux.

L'article de A. Kumar et al. intitulé "A Review of Error-Related Potential-Based Brain-Computer Interfaces for Motor Impaired People" publié dans IEEE Access, vol. 7 , septembre 2019, DOI: 10.1109/ACCESS.2019.2944067 présente un examen de la recherche sur les interfaces neuronales directes pour personnes a motricité diminuée basées sur un potentiel d'erreur.

Une méthode de calibration *on-line* d'une interface BCI a été décrite dans l'article de A. Eliseyev et al. intitulé « Recursive exponentially weighted N-way Partial Least Squares régression with recursive validation of hyper-parameters in Brain-Computer Interface applications » publié dans Scientific Reports, vol. 7, n° 1, p. 16281, nov. 2017, doi:10.1038/s41598-017-16579-9, ainsi que dans la demande de brevet FR-A-3 061 318. Cette méthode sera désignée par la suite sous l'acronyme REW-NPLS (*Recursive Exponentially Weighted N-way Partial Least Squares*).

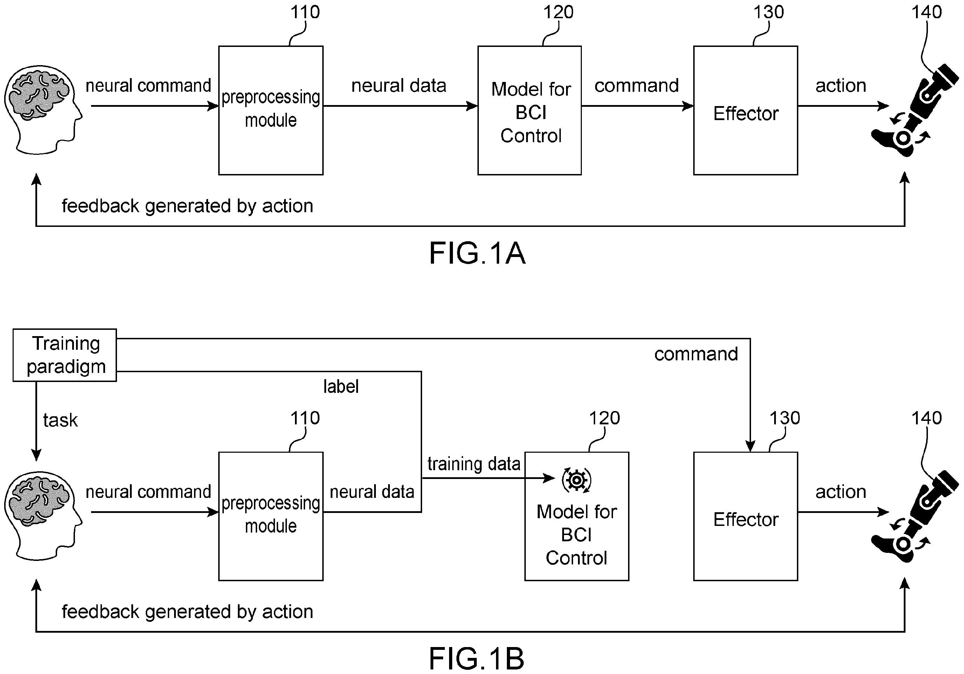

Du fait de la non-stationnarité des signaux neuronaux, des sessions dédiées de calibration en ligne doivent être périodiquement prévues pour entraîner le modèle prédictif de l'interface BCI. Les phases d'utilisation et de calibration de l'interface BCI sont mutuellement exclusives l'une de l'autre comme expliqué en relation avec les Figs. 1A et 1B.

On a représenté schématiquement en Fig. 1A le fonctionnement d'une interface neuronale directe préalablement entraînée.

Les signaux ECoG du sujet sont captés et soumis à un traitement dans un module de prétraitement 110 pour fournir un tenseur de données d'observation d'ordre $P$, noté $\underline{\mathbf{X}}^t$, où t représente un instant d'observation. Le tenseur de données d'observation est généralement de dimension $I_1 \times ... \times I_P$.

Le tenseur d'observation est ensuite fourni comme tenseur d'entrée à un modèle prédictif 120 préalablement entraîné. Ce dernier prédit, à partir du tenseur d'entrée, un tenseur de sortie (ou tenseur de commande) d'ordre $M$, noté $\underline{\mathbf{Y}}^t$. Le tenseur de sortie est généralement de dimension $J_1 \times ... \times J_M$. L'instant $t$ en indice est celui auquel la commande est appliquée, les données de commande pouvant correspondre aux différents effecteurs, représentés schématiquement en 130, ou bien aux différents degrés de liberté d'un robot multiaxe par exemple.

Ces différents effecteurs permettent de mouvoir un exosquelette (ou un robot-multiaxes), représenté en 140. Le mouvement de l'exosquelette engendre une contre-réaction sensorielle (visuelle par exemple) chez le sujet ce qui se traduit par la génération de nouveaux signaux ECoG.

Une session d'entrainement en mode supervisé d'une interface neuronale directe a été schématiquement représentée en Fig. 1B.

On reconnaît sur la figure 1B le module de traitement 110, le modèle prédictif 120, les effecteurs 130 et l'exosquelette 140.

Dans cette phase d'entrainement (ou de calibration) de l'interface BCI, on demande au sujet d'effectuer une tâche prédéterminée (par exemple d'effectuer un mouvement). Les données d'observation (représentées de manière synthétique par le tenseur d'observation) issues du module de traitement sont labellisées par des étiquettes (*labels*) associées aux données de commande (représentées de manière synthétique par un tenseur de commande) correspondant à l'exécution de la tâche par les effecteurs. Ainsi, le module prédictif est entraîné à déduire à partir des données d'observation associées à la tâche en question (variables prédictives) les données de commande permettant de réaliser cette tâches (variables cibles). Le modèle prédictif peut notamment être entraîné au moyen de l'algorithme REW-NPLS cité plus haut.

On comprend par conséquent que les phases d'entrainement ne permettent pas une utilisation libre de l'interface BCI. L'interruption de cette utilisation libre par des phases d'entrainement dédiées est très pénalisante en termes de disponibilité et de praticité.

L'objet de la présente invention est par conséquent de proposer une interface neuronale directe qui puisse s'adapter à la non-stationnarité des signaux neuronaux tout en évitant d'interrompre son utilisation par des phases d'entrainement dédiées.

**EXPOSÉ DE L'INVENTION**

La présente invention est définie par une méthode d'apprentissage d'une interface neuronale directe destinée à recevoir une pluralité de signaux électrophysiologiques exprimant une commande neuronale d'un sujet, pendant une pluralité de fenêtres d'observation associées à des instants d'observation, lesdits signaux électrophysiologiques étant prétraités dans un module de prétraitement pour former en chaque instant d'observation un tenseur de données d'observation, l'interface neuronale directe utilisant un modèle prédictif pour déduire à chaque instant d'observation un tenseur de données de commande à partir du tenseur de données d'observation, lesdites données de commande étant destinées à contrôler au moins un effecteur pour réaliser une trajectoire, ladite méthode d'apprentissage étant originale en ce que :

- on décode à chaque instant d'observation au moyen d'un décodeur préalablement entraîné, un état mental de satisfaction/ erreur du sujet à partir du tenseur de données d'observation, ledit état mental étant représentatif de la conformité de la trajectoire avec la commande neuronale ;
- on génère des données d'apprentissage à partir de l'état mental de satisfaction/erreur décodé en un instant d'observation donné, et du couple formé par le tenseur de données d'observation et le tenseur de données de commande à un instant précédent;
- on met à jour les paramètres du modèle prédictif en minimisant une fonction de coût sur les données d'apprentissage générées à l'étape précédente.

Le décodeur d'état mental est avantageusement entraîné dans une phase préalable en présentant simultanément au sujet une consigne de déplacement et une trajectoire, le tenseur de données d'observation étant labellisé avec un état mental de satisfaction lorsque la trajectoire est conforme à la consigne et avec un état mental d'erreur lorsque celle-ci s'en écarte.

Le décodeur d'état mental fournit typiquement en chaque instant d'observation une prédiction de l'état mental sous la forme d'une valeur binaire ( $\hat{y}^t_{D,mental\_state}$ ) ainsi qu'une estimation du degré de certitude de cette prédiction ( $\left|\hat{y}^t_{mental\_state}\right|$ ).

Selon un premier mode de réalisation, la prédiction effectuée par le modèle prédictif est basée sur une classification, le tenseur de données de commande étant obtenu à partir de la classe la plus probable prédite par le modèle prédictif.

Dans ce cas, si l'état mental prédit en un instant d'observation est un état de satisfaction, les données d'apprentissage peuvent n'être générées à partir du tenseur de données d'observation et du tenseur de données de commande à l'instant d'observation précédent, que si le degré de certitude de l'état mental prédit est supérieur à une première valeur de seuil prédéterminée ( $Th^1_{mental\_state}$ ).

Si l'état mental prédit en un instant d'observation est un état d'erreur, les données d'apprentissage peuvent n'être générées à partir du tenseur de données d'observation et du tenseur de données de commande à l'instant d'observation précédent, que si le degré de certitude de l'état mental prédit est supérieur à une seconde valeur de seuil prédéterminée ( $Th^2_{mental\_state}$ ).

Selon le premier mode de réalisation, si l'état mental prédit en un instant d'observation est un état d'erreur, les données d'apprentissage générées comprennent le tenseur de données d'observation à l'instant d'observation précédent ainsi qu'un tenseur de données de commande obtenu à partir de la seconde classe la plus probable prédite par le modèle prédictif à l'instant d'observation précédent.

La fonction de coût utilisée pour la mise à jour des paramètres du modèle prédictif exprime avantageusement l'écart quadratique entre le tenseur de données de commande prédit par le modèle et celui fourni par les données d'apprentissage, ledit écart quadratique étant pondéré par le degré de certitude prédit par le décodeur d'état mental lors de la génération de ces données d'apprentissage, l'écart quadratique ainsi pondéré étant sommé sur l'ensemble des données d'apprentissage.

Selon un second mode de réalisation, la prédiction effectuée par le modèle prédictif est basée sur une régression linéaire ou multilinéaire.

Selon une première variante, si l'état mental prédit en un instant d'observation est un état d'erreur, les données d'apprentissage ne sont pas générées si cet état mental prédit est un état de satisfaction, les données d'apprentissage ne sont générées à partir du tenseur de données d'observation et du tenseur de données de commande à l'instant d'observation précédent, que si le degré de certitude de l'état mental prédit est supérieur à une première valeur de seuil prédéterminée ( $Th^1_{mental\_state}$ ).

Selon une seconde variante, quel que soit l'état prédit en un instant d'observation, les données d'apprentissage sont générées à partir du tenseur de données d'observation et du tenseur de données de commande à l'instant d'observation précédent, les données d'apprentissage étant alors associées avec le degré de certitude de la prédiction de l'état mental prédit ( $\left|\hat{y}^t_{mental\_state}\right|$ ).

La fonction de coût utilisée pour la mise à jour des paramètres du modèle prédictif dépend avantageusement de l'écart quadratique entre le tenseur de données de commande prédit par le modèle prédictif et celui fourni par les données d'apprentissage, cette dépendance avec l'écart quadratique étant croissante lorsque l'état mental prédit lors de la génération des données d'apprentissage était un état de satisfaction et de manière décroissante lorsque cet état mental était un signal d'erreur, ledit écart quadratique étant pondéré par un facteur dépendant de manière croissante du degré de certitude de l'état mental prédit, associé aux données d'apprentissage.

## BRÈVE DESCRIPTION DES DESSINS

D'autres caractéristiques et avantages de l'invention apparaîtront à la lecture d'un mode de réalisation préférentiel de l'invention, décrit en référence aux figures jointes parmi lesquelles :

La Fig. 1A représente de manière schématique le fonctionnement d'une interface neuronale directe préalablement entraînée ;

La Fig. 1B représente de manière schématique une session d'apprentissage supervisée d'une interface neuronale directe ;

La Fig. 2 représente de manière schématique le fonctionnement d'une interface neuronale directe auto-adaptative selon un mode de réalisation de la présente invention utilisant un premier type d'architecture ;

La Fig. 3 représente de manière schématique le fonctionnement d'une interface neuronale directe auto-adaptative selon un mode de réalisation de la présente invention utilisant un second type d'architecture.

## EXPOSÉ DÉTAILLÉ DE MODES DE RÉALISATION PARTICULIERS

On considérera dans la suite une interface neuronale directe (BCI) telle que présentée dans la partie introductive.

Les signaux électrophysiologiques issus des différentes électrodes sont échantillonnés et rassemblés par blocs de données, chaque bloc correspondant à une fenêtre glissante d'observation de largeur $\Delta T$. Chaque fenêtre d'observation est définie par un instant d'observation ou époque (*epoch*) auquel démarre la fenêtre en question.

Les signaux électrophysiologiques peuvent faire l'objet d'un prétraitement. Ce prétraitement peut notamment comporter une suppression de la moyenne prise sur l'ensemble des électrodes, puis une analyse temps-fréquence est effectuée sur chacune des fenêtres d'observation.

L'analyse temps-fréquence peut être basée sur une décomposition en ondelettes, par exemple en ondelettes de Morlet ou une décomposition CCWT (*Continuous Complex Wavelet Transform*). L'homme du métier comprendra toutefois que d'autres types d'analyse temps-fréquence pourront être envisagés par l'homme du métier.

Ces résultats de l'analyse temps-fréquence peuvent en outre faire l'objet d'un lissage fréquentiel ou d'une décimation.

Ainsi, à chaque fenêtre d'observation, ou instant d'observation $t$, est associé un tenseur d'ordre 3 de données d'observation, noté $\underline{\mathbf{X}}^t$, dont le premier mode correspond aux positions temporelles des ondelettes, le deuxième mode correspond à la fréquence, autrement dit au nombre de bandes de fréquence utilisées pour la décomposition en ondelettes sur une fenêtre d'observation et le quatrième mode correspond à l'espace, autrement dit aux capteurs (électrodes).

Ainsi, $\underline{\mathbf{X}}^t \in \mathbb{R}^{\tau \times f \times s}$ et le tenseur complet des données d'observation, c'est-à-dire l'historique des observations, est noté $\underline{\mathbf{X}} \in \mathbb{R}^{N \times \tau \times f \times s}$ où $N$ est le nombre d'époques, $\tau$ est le nombre de positions temporelles des ondelettes (*temporal features*), le cas échéant après moyennage sur une pluralité de positions temporelles successives, $f$ est le nombre de bandes de fréquences (*frequency features*), et $s$ est le nombre de capteurs (*spatial features*). De manière plus générale, le tenseur des données observation, $\underline{\mathbf{X}}^t$, relatif à l'époque t pourra être d'ordre $P$. Dans ce cas, le tenseur d'observation $\mathbf{X}^t$ est de dimension $I_1 \times ... \times I_P$. Toutefois, sans perte de généralité, l'invention sera décrite dans le cas $P = 3$ précité.

De la même façon, la trajectoire du mouvement imaginé, observé ou réalisé à l'instant $t$ pourra être décrite par un tenseur de sortie (ou tenseur de commande) d'ordre $Q$, noté $\underline{\mathbf{Y}}^t$, de dimension $J_1 \times ... \times J_Q$, dont les différents modes correspondant aux commandes de différents effecteurs (ou aux différents degrés de liberté d'un robot multiaxe).

Plus précisément, le tenseur de sortie fournit des blocs de données de commande, chacun des blocs permettant de générer les signaux de commande relatifs aux différents effecteurs ou degrés de liberté. Ainsi, on comprendra que la dimension de chaque bloc de données pourra dépendre du cas d'usage envisagé et notamment du nombre de degrés de liberté de l'effecteur. Sans perte de généralité, on supposera dans la suite que le tenseur de commande $\underline{\mathbf{Y}}^t$ est d'ordre $Q = 1$. Autrement dit, $\underline{\mathbf{Y}}^t \in \mathbb{R}^M$ où $M$ est le nombre de degrés de liberté de la commande (ou de l'effecteur).

Le modèle prédictif permettant de passer du tenseur d'observation au tenseur de commande peut être basé sur une classification et/ou une régression. Dans le cas d'une classification, le tenseur de commande peut indiquer par exemple une direction de mouvement (gauche, droite, avant, arrière), dans le cas d'une régression le tenseur de commande pourra donner les données de commande des différents effecteurs.

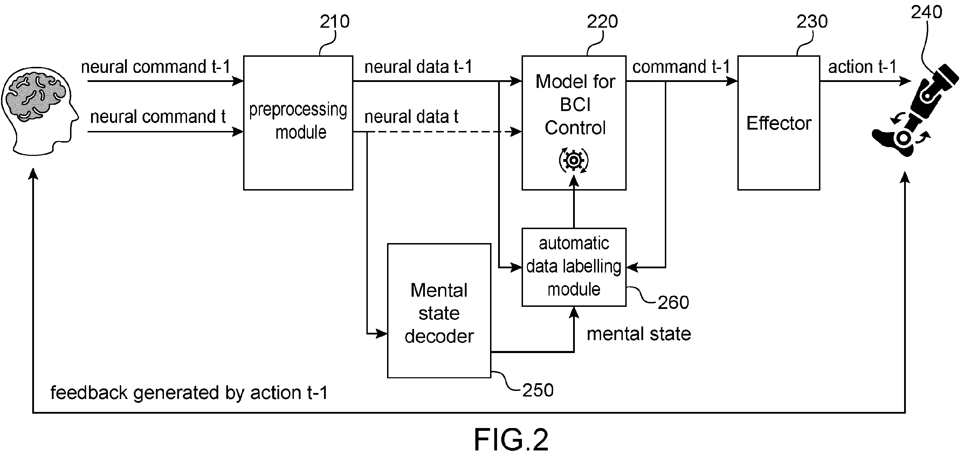

La Fig. 2 représente de manière schématique le fonctionnement d'une interface neuronale directe auto-adaptative selon un mode de réalisation de la présente invention utilisant un premier type d'architecture.

Les éléments portant les références 210 à 240 sont identiques aux éléments 110 à 140 précédemment décrits. L'interface BCI représentée comprend en outre un décodeur d'état mental 250, préalablement entraîné, recevant le tenseur de données d'observation, $\mathbf{X}^t$ à l'époque $t$ et estimant à partir de ce tenseur un vecteur d'état représentatif de l'état mental du sujet en ce même instant. Par état mental en un instant t, on entend ici un état de satisfaction ou un état d'erreur détecté à partir des signaux électrophysiologiques (typiquement des signaux ECoG) recueillis par des électrodes placées sur le cortex moteur du sujet. Plus précisément, cet état mental indique si le sujet est satisfait ou non de l'évolution de la trajectoire (d'un curseur, d'un effecteur ou d'un robot multiaxe par exemple) autrement dit si le tenseur de commande $\mathbf{Y}^{t\text{-}1}$ produit par le modèle prédictif et appliqué à l'effecteur à l'instant $t$-1 est bien conforme à la trajectoire de consigne souhaitée par le sujet.

Il est important de bien faire ici la distinction entre le décodage d'un état mental d'erreur dans le contexte de la présente invention, d'une part, et la détection d'un potentiel d'erreur, d'autre part. Un potentiel d'erreur ou signal ErrP (*error-related potential*) est un signal cérébral observé en réponse à un évènement discret, par exemple une action ponctuelle erronée. Autrement dit, un tel signal ErrP est déclenché par une erreur intervenue en un instant donné et ne résulte pas d'une action continue telle qu'une déviation observée au cours du temps par rapport à une trajectoire de consigne. En pratique, les signaux ErrP se manifestent sous la forme d'une déflexion négative de potentiel dans une zone fronto-centrale du cuir chevelu (apparaissant environ 50 à 100 ms après la survenance de l'évènement discret), suivie d'une déflexion positive de potentiel dans la zone fronto-pariétale. Ils peuvent être enregistrés par de simples électrodes cutanées placées sur le cuir chevelu alors que les signaux ECoG sont obtenus à partir d'électrodes situées sur le cortex cérébral moteur du sujet.

Le décodeur d'état mental est entraîné de manière supervisée dans une phase préalable distincte de la phase d'utilisation de l'interface BCI. Dans cette phase d'entraînement le sujet pourra, par exemple, se voir présenter simultanément une consigne de déplacement ainsi qu'une trajectoire. Dans le même temps, les tenseurs de données d'observation en sortie du module de prétraitement sont stockés dans une base de données d'apprentissage d'état mental.

Si la trajectoire est conforme à (ou tend à se rapprocher de) la consigne, le tenseur de données d'observation est labellisé comme un état mental de satisfaction de la consigne. A l'inverse, lorsque la trajectoire est non conforme à (ou tend à s'écarter de) la consigne, le tenseur de données d'observation est labellisé comme un état mental d'erreur. Dans un exemple de réalisation particulièrement simple, la consigne pourra être une commande de marche/arrêt. Ainsi, un avatar pourra être montré sur un écran simultanément avec un symbole indiquant la consigne. Si l'avatar marche alors que la consigne est une instruction d'arrêt ou bien si l'avatar est immobile alors que la consigne est une instruction de marche, les tenseurs de données d'observation correspondants sont labellisés avec une étiquette d'état mental d'erreur. En revanche, si l'avatar marche et s'arrête selon les instructions données par la consigne, les tenseurs de données d'observation sont labellisées avec une étiquette d'état mental de satisfaction. Bien entendu, d'autres types d'apprentissage d'état mental pourront être envisagés par l'homme du métier selon la nature de la commande, sans pour autant sortir du cadre de la présente invention, tel que défini par les revendications.

Ainsi la consigne pourra être une instruction de direction (gauche, droite, avant, arrière) ou encore une instruction indiquant le membre à mouvoir (pied gauche, pied droit, main droite, main gauche). Lorsque le mouvement de l'avatar est conforme à l'instruction donnée par la consigne, l'étiquette d'état mental associée aux tenseurs de données d'observation correspond à un état mental de satisfaction. A défaut, lorsque le mouvement de l'avatar diffère de l'instruction donnée par la consigne, l'étiquette mental associée tenseurs de données d'observation correspond à un état mental d'erreur.

L'état mental de satisfaction/erreur à l'instant $t$ peut être représenté par une valeur binaire signée ou un booléen (classifieur dans 2 classes), noté $y^{t}_{D,mental\_state}$ . L'ensemble des données d'apprentissage (*training data set*) du décodeur d'état mental est alors constitué par les couples ( $(\tilde{\underline{\mathbf{X}}}^{t}, \tilde{y}^{t}_{D,mental\_state})$ ) en une pluralité d'instants d'observation $t$ (le signe tilde indique le fait qu'il s'agisse de données d'apprentissage).

Le décodeur d'état d'erreur peut être par exemple implémenté au moyen d'un réseau de neurones artificiels ou un classifieur SVM, voire d'un algorithme de type NPLS.

Après sa phase d'apprentissage, le décodeur d'état mental 250 peut prédire l'état mental de satisfaction/erreur à partir d'un tenseur de données d'observation, $\underline{\mathbf{X}}^{t}$.

Selon une première variante, l'état mental de satisfaction/ erreur prédit à l'instant $t$ par le décodeur 250 se présente sous la forme d'une valeur binaire, notée $\hat{y}^{t}_{D,mental\_state}$ . Par exemple, un état mental de satisfaction sera indiqué par $\hat{y}^{t}_{D,mental\_state} = 1$ et un état mental d'erreur sera indiqué par $\hat{y}^{t}_{D,mental\_state} = -1$ .

Selon une seconde variante, l'état mental de satisfaction/ erreur prédit à l'instant $t$ par le décodeur 250 se présente sous la forme d'une valeur réelle, notée indiquant la probabilité que l'état mental à une classe plutôt qu'à une autre. Par exemple, la valeur réelle peut être un logarithme de rapport de la probabilité d'appartenir à une classe plutôt qu'à une autre. Ainsi, une valeur positive de $\hat{y}^{t}_{mental\_state}$ pourra traduire un état mental de satisfaction et une valeur négative $\hat{y}^{t}_{mental\_state}$ pourra alors traduire un état mental d'erreur, le degré de certitude de la prévision étant donné dans les deux cas par $\left| \hat{y}^{t}_{mental\_state} \right|$ .

Lors de l'utilisation de l'interface BCI, le décodeur d'état mental peut fournir à chaque époque $t$ une prédiction de l'état mental du sujet à partir du tenseur de données d'observation $\mathbf{X}^{t}$. Cette prédiction d'état mental est utilisée par un module de labellisation automatique de données, 260, pour construire à la volée de nouvelles données d'apprentissage à partir du couple formé par le tenseur de données d'observation et du tenseur de données de commande à l'époque précédente, à savoir $(\mathbf{X}^{t-1}, \mathbf{Y}^{t-1})$.

Cette création de données d'apprentissage n'est en général pas systématique à chaque époque mais peut intervenir lors de phases d'apprentissage intervenant de manière périodique ou bien asynchrone. Sans perte de généralité, on supposera qu'une phase d'apprentissage d'indice $u$ commence à l'époque $n(u-1)+1$ et se termine à l'époque $nu$. Les données d'observation peuvent être représentées par les tenseurs d'observation $\mathbf{X}^{t}$ aux instants consécutifs $t$ $=n(u-1)+1,...nu$ et donc par un tenseur $\underline{\mathbf{X}}^{u}$ d'ordre $P+1=4$, $\underline{\mathbf{X}}^{u} \in \mathbb{R}^{n\times\tau\times f\times s}$ , tel que $\underline{\mathbf{X}}^{u}_{(1)} = \underline{\mathbf{X}}^{t}$ , $t=n(u-1)+1,...nu$ où $\underline{\mathbf{X}}^{u}_{(1)}$ représente le déploiement de $\underline{\mathbf{X}}^{u}$ selon le premier mode. De manière similaire, les données de commande en ces mêmes instants peuvent être représentées par un tenseur d'ordre $Q+1=2$, $\underline{\mathbf{Y}}^{u} \in \mathbb{R}^{n\times M}$ tel que $\underline{\mathbf{Y}}^{u}_{(1)} = \underline{\mathbf{Y}}^{t}$ , $t=n(u-1)+1,...nu$ où $\underline{\mathbf{Y}}^{u}_{(1)}$ représente le déploiement de $\underline{\mathbf{Y}}^{u}$ selon le premier mode. Enfin, on représente par $\hat{\mathbf{y}}^{u}_{mental\_state}$ le tenseur d'ordre 1, autrement dit le vecteur de $\mathbb{R}^{n}$ dont les éléments sont $\hat{y}^{t}_{mental\_state}$ , $t = n(u-1)+1,..nu$ .

De manière générale, à chaque phase d'apprentissage, le module de labellisation automatique construit de manière automatique des données d'apprentissage définies par le couple $(\tilde{\mathbf{X}}^{u}, \tilde{\mathbf{Y}}^{u})$ , tel que :

$$ \tilde{\underline{\mathbf{Y}}}^{u} = \Phi\left( \underline{\mathbf{Y}}^{u}, \hat{\mathbf{y}}^{u}_{mental\_state} \right) \tag{1-1} $$

$$ \tilde{\underline{\mathbf{X}}}^{u} = \underline{\mathbf{X}}^{u} \tag{1-2} $$

où $\Phi$ est une application de $\mathbb{R}^{n\times M} \times \mathbb{R}^{n} \rightarrow \mathbb{R}^{n\times M}$ . Plus précisément, pour construire de manière automatique des données d'apprentissage, le module de labellisation automatique utilise les tenseurs de données d'observation de la phase $u$ et leur associe des tenseurs de données de commande modifiés par la fonction $\Phi$ lorsque les états mentaux observés pendant cette phase comprennent au moins un état mental d'erreur. Plus précisément, la modification d'un tenseur de commande à une époque $t_c$ de la phase $u$ pourra dépendre des états mentaux prédits aux instants $t_c +1,...,nu + 1$, voire également des états antérieurs à $t_c$.

Sans perte de généralité, on supposera dans la suite qu'un tenseur de commande à une époque t ne dépend que de l'état mental prédit à l'instant suivant $t + 1$. Autrement dit, lorsque le sujet reçoit un feedback sensoriel à l'instant $t + 1$ (correction ou erreur de la trajectoire) après que le tenseur de données de commande a été appliqué, le module de labellisation modifie (ou corrige) le tenseur de données de commande relatif à l'instant précédent, $t$, ce qui peut s'exprimer par :

$$ \tilde{\underline{\mathbf{X}}}^{t} = \underline{\mathbf{X}}^{t} \tag{2-1} $$

$$ \tilde{\underline{\mathbf{Y}}}^{t} = \varphi\left( \underline{\mathbf{Y}}^{t}, \hat{y}^{t+1}_{mental\_state} \right) \quad t = n(u-1)+1,...nu \tag{2-2} $$

où $\varphi$ est une application de $\mathbb{R}^{M} \times \mathbb{R} \rightarrow \mathbb{R}^{M}$ .

L'application $\varphi$ (ou plus généralement l'application $\Phi$) peut prendre différentes formes en fonction du type de prédiction effectuée par le modèle prédictif 210. Dans tous les cas, son objet est de mettre à jour l'ensemble des données d'apprentissage avec le couple ($\underline{\mathbf{X}}^t$, $\underline{\tilde{\mathbf{Y}}}^t$) grâce à l'état mental de satisfaction/erreur prédit, $\hat{y}^{t+1}_{mental\_state}$ à au moins l'instant suivant de la phase d'apprentissage. Si l'état mental prédit pour au moins cet instant suivant correspond à un état mental d'erreur, le tenseur de données de commande $\underline{\mathbf{Y}}^t$ est corrigé par l'application $\varphi$ pour générer les nouvelles données d'apprentissage ($\underline{\mathbf{X}}^t$, $\tilde{\mathbf{Y}}^t$). En revanche, si tous les états mentaux postérieurs $\hat{y}^{t+1}_{mental\_state}, \ldots, \hat{y}^{nu+1}_{mental\_state}$ sont tous des états de satisfaction, le couple ($\underline{\mathbf{X}}^t$, $\mathbf{Y}^t$) peut être incorporé tel quel dans l'ensemble de données d'apprentissage.

Selon un premier mode de réalisation, la prédiction effectuée par le modèle prédictif est basée sur une opération de classification fournissant un vecteur $\mathbf{y}^t_{class} = \left( y^t_1, y^t_2, \ldots, y^t_M \right)^T$ des probabilités d'appartenance à $M$ classes possibles, et le vecteur de commande ($\underline{\mathbf{Y}}^t$) fourni par le modèle prédictif est donné par :

$$\mathbf{y}^t_{control} = \mathbf{e}_{m^t_1} \tag{3}$$

où $m^t_1 = \underset{m=1,\ldots,M}{\arg\max}\left( y^{t+1}_m \right)$ et $(\mathbf{e}_m)_{m=1,\ldots,M}$ est la base canonique de $\mathbb{R}^M$. Autrement dit le vecteur de commande correspond à la classe de plus forte probabilité.

Le module de labellisation automatique met à jour l'ensemble de données d'apprentissage en y incorporant le couple ($\underline{\tilde{\mathbf{X}}}^t, \underline{\tilde{\mathbf{Y}}}^t$) = ($\underline{\tilde{\mathbf{X}}}^t, \tilde{\mathbf{y}}^t$) défini par :

$$\underline{\tilde{\mathbf{X}}}^t = \underline{\mathbf{X}}^t \tag{4-1}$$

$$\tilde{\mathbf{y}}^t = \varphi\left( \mathbf{y}^t_{control}, \hat{y}^{t+1}_{mental\_state} \right) \tag{4-2}$$

où :

$$\varphi\left( \mathbf{y}^t_{control}, \hat{y}^{t+1}_{mental\_state} \right) = \mathbf{y}^t_{control} \tag{5-1}$$

si l'état mental est un état mental de satisfaction ( $\hat{y}^{t+1}_{D,mental\_state} = +1$ ou $\hat{y}^{t+1}_{mental\_state} > 0$ ) ; et

$$\varphi\left( \mathbf{y}^t_{control}, \hat{y}^{t+1}_{mental\_state} \right) = \mathbf{T}_{m^t_1 m^t_2} \mathbf{y}^t_{control} = \mathbf{e}_{m^t_2} \tag{5-2}$$

si l'état mental est un état mental d'erreur ( $\hat{y}^{t+1}_{D,mental\_state} = -1$ ou $\hat{y}^{t+1}_{mental\_state} < 0$ ), $\mathbf{T}_{m^t_1 m^t_2}$ étant la matrice de taille $M \times M$ permutant les lignes $m^t_1$ et $m^t_2$ avec $m^t_2 = \underset{\substack{m=1,\ldots,M \\ m \neq m^t_1}}{\arg\max}\left( y^{t+1}_m \right)$. En d'autres termes, si l'état mental à l'instant suivant est un état d'erreur, le vecteur de commande est donné par la seconde classe la plus probable.

Selon une variante, l'incorporation de nouvelles données d'apprentissage est sélective. Plus précisément, dans ce cas, un couple ($\underline{\tilde{\mathbf{X}}}^t, \underline{\tilde{\mathbf{Y}}}^t$) ne sera incorporé dans $\Omega^u$ que dans la mesure où le degré de certitude de l'état mental de satisfaction en (5-1) excède une valeur de seuil prédéterminée, c'est-à-dire si $\hat{y}^{t+1}_{mental\_state} > Th^1_{mental\_state} > 0$. De manière similaire, la correction effectuée en (5-2) pourra également être sélective et n'être réalisée que dans la mesure

$$\hat{y}^{t+1}_{mental\_state} < Th^2_{mental\_state} < 0$$

où $Th^2_{mental\_state}$ est une seconde valeur de seuil prédéterminée.

Le modèle prédictif 220 est mis à jour au moyen des nouvelles données d'apprentissage fournies par le module de labellisation automatique. Cette mise à jour n'intervient pas nécessairement aussitôt que ces nouvelles données d'apprentissage sont disponibles. En effet, ces dernières peuvent être stockées localement pour une mise à jour ultérieure, réalisée périodiquement ou bien dès que le nombre de nouvelles données d'apprentissage atteint un seuil prédéterminé.

La mise à jour des paramètres du modèle prédictif est réalisée en minimisant une fonction de coût donnant l'écart quadratique entre les prédictions du modèle et les étiquettes pour les données de l'ensemble d'apprentissage, soit :

$$\Theta^u = \arg\min_{\Theta} \sum_{\left(\underline{\tilde{\mathbf{X}}}^t, \underline{\tilde{\mathbf{Y}}}^t\right) \in \Omega^u} \left\| F\left(\underline{\tilde{\mathbf{X}}}^t; \Theta\right) - \underline{\tilde{\mathbf{Y}}}^t \right\|^2 \tag{6}$$

où $\Theta$ désigne l'ensemble des paramètres du modèle prédictif, $\Theta^u$ désigne l'ensemble des paramètres minimisant la fonction de coût lors de la mise à jour $u$, $F(.;\Theta)$ est la fonction de prédiction du modèle dépendant de l'ensemble de paramètres $\Theta$, $\Omega^u = \Omega^{u-1} \cup \{(\underline{\tilde{\mathbf{X}}}^t, \underline{\tilde{\mathbf{Y}}}^t); t=(u-1)n+1,...,un\}$ est l'ensemble des données d'apprentissage lors de la mise à jour $u$.

La fonction de coût pourra faire intervenir un poids fonction du degré de certitude de la prédiction d'état mental pour pondérer l'écart quadratique de prédiction du tenseur de commande, soit :

$$\Theta^u = \arg\min_{\Theta} \sum_{\left(\underline{\tilde{\mathbf{X}}}^t, \underline{\tilde{\mathbf{Y}}}^t\right) \in \Omega^u} w\left(\hat{y}^{t+1}_{mental\_state}\right) \left\| F\left(\underline{\tilde{\mathbf{X}}}^t; \Theta\right) - \underline{\tilde{\mathbf{Y}}}^t \right\|^2 \tag{7}$$

où $w\left(\hat{y}^{t+1}_{mental\_state}\right)$ est une fonction croissante du degré de certitude pour $\hat{y}^{t+1}_{mental\_state} > 0$, autrement dit lorsque l'état mental est un état mental de satisfaction. En d'autres termes, la fonction de coût donnera plus de poids aux données d'apprentissage présentant une probabilité plus élevée de correspondre à un état mental de satisfaction.

On pourra prendre par exemple :

$$w\left(\hat{y}^{t+1}_{mental\_state}\right) = \max\left(\hat{y}^{t+1}_{mental\_state} - Th^1_{mental-state}, 0\right) \tag{8-1}$$

ou bien

$$w\left(\hat{y}^{t+1}_{mental\_state}\right) = h\left(\hat{y}^{t+1}_{mental\_state} - Th^1_{mental-state}\right) \tag{8-2}$$

où $h(.)$ est l'échelon de Heaviside. On remarquera que le choix d'une fonction de pondération selon (8-2) est équivalent à l'incorporation sélective dans l'ensemble d'apprentissage selon la variante précitée.

Selon un second mode de réalisation, la prédiction effectuée par le modèle prédictif est basée sur une opération de régression, par exemple une régression linéaire ou multilinéaire :

$$\underline{\mathbf{Y}}^t = \underline{\mathbf{B}}\underline{\mathbf{X}}^t + \underline{\boldsymbol{\beta}} \tag{9}$$

où $\underline{\mathbf{B}}$ est un tenseur de coefficients de prédiction $\underline{\mathbf{B}} \in \mathbb{R}^{(I_1 \times ... \times I_P) \times (J_1 \times ... \times J_Q)}$ et $\underline{\beta}$ est un tenseur de biais $\underline{\boldsymbol{\beta}} \in \mathbb{R}^{(J_1 \times ... \times J_M)}$. L'ensemble des paramètres du modèle prédictif est ici constitué par les coefficients des tenseurs $\underline{\mathbf{B}}$ et $\underline{\beta}$, $\Theta = \{\underline{\mathbf{B}}, \underline{\beta}\}$. Le tenseur $\underline{\mathbf{Y}}^t$ fourni par le modèle prédictif est utilisé directement pour la commande.

Alternativement, la prédiction pourra être réalisée au moyen d'une régression non-linéaire par exemple au moyen d'un réseau de neurones artificiels.

Quel que soit le type de régression, selon une première variante, le module de labellisation automatique met à jour l'ensemble de données d'apprentissage $\Omega^u$ en y incorporant le couple $(\underline{\tilde{\mathbf{X}}}^t, \underline{\tilde{\mathbf{Y}}}^t) = (\underline{\mathbf{X}}^t, \underline{\mathbf{Y}}^t)$ si le degré de certitude de l'état mental de satisfaction excède une valeur de seuil prédéterminée ( $\hat{y}^t_{mental\_state} > Th^1_{mental\_state}$ ). A défaut, la labellisation n'est pas effectuée et le couple $(\underline{\mathbf{X}}^t, \underline{\mathbf{Y}}^t)$ n'est pas incorporé dans $\Omega^u$.

Comme dans le premier mode de réalisation, la mise à jour des paramètres du modèle prédictif pourra être réalisée en minimisant une fonction de coût donnant l'écart quadratique entre les prédictions du modèle et les étiquettes pour les données de l'ensemble d'apprentissage :

$$\Theta^u = \arg \min_{\Theta} \sum_{\left(\underline{\tilde{\mathbf{X}}}^t, \underline{\tilde{\mathbf{Y}}}^t\right) \in \Omega^u} \left\| F\left(\underline{\tilde{\mathbf{X}}}^t; \Theta\right) - \underline{\tilde{\mathbf{Y}}}^t \right\|^2 \tag{10}$$

où $F(.;\Theta)$ est la fonction de régression.

Selon une seconde variante de réalisation, le module de labellisation automatique met à jour l'ensemble de données d'apprentissage $\Omega^u$ en y incorporant le couple $(\underline{\tilde{\mathbf{X}}}^t, \underline{\tilde{\mathbf{Y}}}^t) = (\underline{\mathbf{X}}^t, \underline{\mathbf{Y}}^t)$ que l'état mental prédit soit un état mental de satisfaction ou un état d'erreur. Dans ce cas, la mise à jour des paramètres sur l'ensemble d'apprentissage se fait par minimisation d'une fonction de coût donnant plus de poids aux données d'apprentissage qui correspondent à un état mental présentant un degré de certitude plus élevé (que cet état mental soit un état mental de satisfaction ou d'erreur) qu'aux données d'apprentissage pour lesquelles l'état mental prédit est incertain, à savoir :

$$\Theta^u = \arg \min_{\Theta} \sum_{\left(\underline{\tilde{\mathbf{X}}}^t, \underline{\tilde{\mathbf{Y}}}^t\right) \in \Omega^u} \exp\left(\hat{y}^{t+1}_{D,mental\_state} . w\left(\hat{y}^{t+1}_{mental\_state}\right). \left\| F\left(\underline{\tilde{\mathbf{X}}}^t; \Theta\right) - \underline{\tilde{\mathbf{Y}}}^t \right\|^2\right)$$

$$\tag{11}$$

avec :

$$w\left(\hat{y}^{t+1}_{mental\_state}\right) = \left| \hat{y}^{t+1}_{mental\_state} \right| \quad \text{si} \quad \hat{y}^{t+1}_{mental\_state} \leq Th_- \text{ ou } \hat{y}^{t+1}_{mental\_state} \geq Th_+ \tag{12-1}$$

et

$$w\left(\hat{y}^{t+1}_{mental\_state}\right) = 0 \quad \text{si} \quad Th_- < \hat{y}^{t+1}_{mental\_state} < Th_+ \tag{12-2}$$

où $Th_-$ et $Th_+$ sont respectivement une valeur de seuil négative et une valeur de seuil positive.

Du fait de la présence de la valeur binaire signée, $\hat{y}^{t+1}_{D,mental\_state}$, dans l'expression (11), la minimisation de la fonction de coût tend à réduire l'écart quadratique de la prédiction sur les données d'apprentissage correspondant à un état mental de satisfaction et à accroître cet écart sur les données d'apprentissage correspondant à un état mental d'erreur. La contribution à la réduction ou à l'accroissement de l'écart quadratique est fonction du degré de certitude de la prédiction de l'état mental, $\left| \hat{y}^{t+1}_{mental\_state} \right|$.

De manière équivalente, la prise en compte d'un poids nul dans l'expression (12-2) peut être mise en oeuvre en n'incorporant dans l'ensemble de données d'apprentissage que les couples de tenseurs $(\underline{\mathbf{X}}^t, \underline{\mathbf{Y}}^t)$ pour lesquels le degré de certitude de l'état mental prédit, $\left| \hat{y}^{t+1}_{mental\_state} \right|$, est suffisamment élevé.

La mise à jour des paramètres du modèle dépend du type de modèle prédictif. Par exemple, si le modèle prédictif est réalisé au moyen d'un réseau de neurones la mise à jour des paramètres peut être classiquement obtenue par rétropropagation du gradient. Lorsque le modèle prédictif est basé sur une régression linéaire ou multilinéaire, la mise à jour des paramètres peut être effectuée selon l'algorithme REW-PLS (*Recursive Exponentially Weigthed Partial Least Squares*) ou REW-NPLS (*Recursive Exponentially Weigthed N-way Partial Least Squares*), la minimisation de fonction de coût étant alors appliquée à chaque étape de la méthode des moindres carrés alternés ou ALS (*Alternate Least Squares*) de la décomposition PARAFAC.

On pourra trouver une description des algorithmes REW-PLS et REW-NPLS dans l'article de A. Eliseyev et al. intitulé « Recursive exponentially weighted N-way Partial Least Squares régression with recursive validation of hyper-parameters in Brain-Computer Interface applications » publié dans Scientific Reports, vol. 7, n° 1, p. 16281, nov. 2017 ainsi que dans la demande de brevet FR-A-3 061318. Ces algorithmes sont avantageux dans la mesure où ils ne nécessitent pas de stocker l'historique des données d'apprentissage mais seulement celles qui ont été labellisées depuis la dernière mise à jour.

Dans le mode de réalisation de la Fig. 2, le module 220 implémentant le modèle prédictif, c'est-à-dire calculant le tenseur de commande à partir du tenseur d'observation est également chargé de la mise à jour des paramètres du modèle. A cette fin, elle stocke localement les données d'apprentissage fournies par le module de labellisation automatique 260. La mise à jour des paramètres peut être réalisée en parallèle avec le calcul du tenseur de commande, par multithreading dans une unité centrale (CPU).

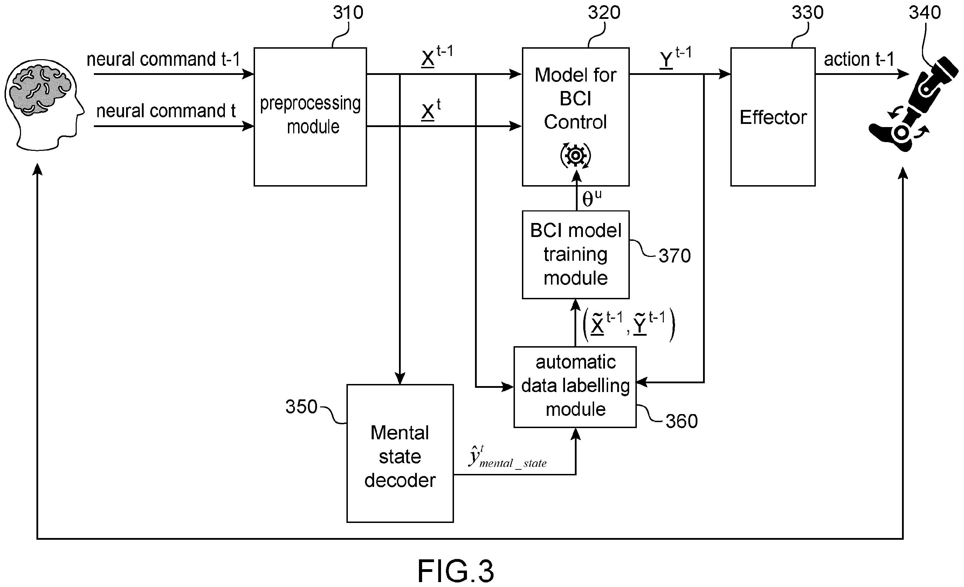

La Fig. 3 représente de manière schématique le fonctionnement d'une interface neuronale directe auto-adaptative selon un mode de réalisation de la présente invention utilisant un second type d'architecture.

Les éléments 310, 330, 340, 350 et 360 sont respectivement identiques aux éléments 210, 230, 240, 250 et 260 de la Fig. 2 et leur description ne sera donc pas reprise ici.

L'interface neuronale directe auto-adaptative de la Fig. 3 diffère de celle de la Fig. 2 en ce qu'elle comprend un module d'entrainement du modèle prédictif, 370, distinct du module implémentant le modèle prédictif lui-même. Autrement dit, le module 320 effectue une prédiction (classification ou régression) du tenseur de données de commande $\underline{\mathbf{Y}}^t$ à partir du tenseur de données d'observation $\underline{\mathbf{X}}^t$ au moyen de la fonction de prédiction $F(.;\Theta)$ mais n'effectue pas lui-même la mise à jour des paramètres $\Theta$. Celle-ci est déléguée au module d'entrainement 370 qui reçoit les données d'apprentissage du module de labellisation automatique 360. Par exemple, lorsqu'un nouveau jeu de paramètres $\Theta^u$ est disponible au terme d'une nouvelle phase d'apprentissage $u$, le module d'entrainement le signale au module de prédiction au moyen d'une interruption à son CPU. Le module de prédiction peut alors télécharger le nouveau jeu de paramètres sans perturbation aucune de la commande.

L'homme du métier comprendra que l'interface neuronale directe décrite ci-dessus est auto-adaptative dans la mesure où elle s'adapte à la non-stationnarité des signaux neuronaux. Elle ne nécessite pas de phase d'entrainement dédiée, l'entrainement pouvant être réalisée sur des données d'apprentissage obtenues par un procédé de labellisation automatique utilisant une prédiction de l'état mental de satisfaction/erreur de l'utilisateur. En outre, les données labélisées correspondent à des tâches qu'effectue réellement l'utilisateur et non à des tâches qui lui sont imposées lors d'un apprentissage supervisé. Enfin, il convient de remarquer la labellisation automatique des données d'observation permet de générer des quantités importantes de données labélisées qui peuvent être utilisées dans une méthode d'apprentissage *off-line.* On peut ainsi obtenir des bases de données d'entrainement par *crowd-sourcing* sans nécessité de longues séances d'entrainement coûteuses et éprouvantes pour l'utilisateur.

**Revendications**

1. Méthode d'apprentissage d'une interface neuronale directe destinée à recevoir une pluralité de signaux électrophysiologiques exprimant une commande neuronale d'un sujet, pendant une pluralité de fenêtres d'observation associées à des instants d'observation, lesdits signaux électrophysiologiques étant prétraités dans un module de prétraitement (210,310) pour former en chaque instant d'observation un tenseur de données d'observation, l'interface neuronale directe utilisant un modèle prédictif (220,320) pour déduire à chaque instant d'observation un tenseur de données de commande à partir du tenseur de données d'observation, lesdites données de commande étant destinées à contrôler au moins un effecteur (230 ,330) pour réaliser une trajectoire, **caractérisée en ce que** :

   - on décode (250,350) à chaque instant d'observation au moyen d'un décodeur préalablement entraîné, un état mental de satisfaction ou d'erreur du sujet (250,350) à partir du tenseur de données d'observation, ledit état mental étant indicatif de la conformité de la trajectoire avec la commande neuronale ;
   - on génère (260, 360) des données d'apprentissage à partir de l'état mental de satisfaction ou d'erreur, décodé en un instant d'observation donné, et du couple formé par le tenseur de données d'observation et le tenseur de données de commande à un instant précédent;
   - on met à jour (220,370) les paramètres du modèle prédictif en minimisant une fonction de coût sur les données d'apprentissage générées à l'étape précédente.

2. Méthode d'apprentissage d'une interface neuronale directe selon la revendication 1, **caractérisée en ce que** le décodeur d'état mental est entraîné dans une phase préalable en présentant simultanément au sujet une consigne de déplacement et une trajectoire, le tenseur de données d'observation étant labellisé avec un état mental de satisfaction lorsque la trajectoire est conforme à la consigne et avec un état mental d'erreur lorsque celle-ci s'en écarte.

3. Méthode d'apprentissage d'une interface neuronale directe selon la revendication 2, **caractérisée en ce que** le décodeur d'état mental fournit en chaque instant d'observation une prédiction de l'état mental sous la forme d'une valeur binaire ($\hat{y}^t_{D,mental\_state}$) ainsi qu'une estimation du degré de certitude de cette prédiction ($\left|\hat{y}^t_{mental\_state}\right|$).

4. Méthode d'apprentissage d'une interface neuronale directe selon la revendication 3, **caractérisée en ce que** la prédiction effectuée par le modèle prédictif est basée sur une classification, le tenseur de données de commande étant obtenu à partir de la classe la plus probable prédite par le modèle prédictif.

5. Méthode d'apprentissage d'une interface neuronale directe selon la revendication 4, **caractérisée en ce que** si l'état mental prédit en un instant d'observation est un état de satisfaction, les données d'apprentissage ne sont générées à partir du tenseur de données d'observation et du tenseur de données de commande à l'instant d'observation précédent, que si le degré de certitude de l'état mental prédit est supérieur à une première valeur de seuil prédéterminée ($Th^1_{mental\_state}$).

6. Méthode d'apprentissage d'une interface neuronale directe selon la revendication 4 ou 5, **caractérisée en ce que** si l'état mental prédit en un instant d'observation est un état d'erreur, les données d'apprentissage ne sont générées à partir du tenseur de données d'observation et du tenseur de données de commande à l'instant d'observation précédent, que si le degré de certitude de l'état mental prédit est supérieur à une seconde valeur de seuil prédéterminée ($Th^2_{mental\_state}$).

7. Méthode d'apprentissage d'une interface neuronale directe selon l'une des revendications 4 à 6, **caractérisée en ce que** si l'état mental prédit en un instant d'observation est un état d'erreur, les données d'apprentissage générées comprennent le tenseur de données d'observation à l'instant d'observation précédent ainsi qu'un tenseur de données de commande obtenu à partir de la seconde classe la plus probable prédite par le modèle prédictif à l'instant d'observation précédent.

8. Méthode d'apprentissage d'une interface neuronale directe selon l'une des revendications 4 à 7, **caractérisée en ce que** la fonction de coût utilisée pour la mise à jour des paramètres du modèle prédictif exprime l'écart quadratique entre le tenseur de données de commande prédit par le modèle et celui fourni par les données d'apprentissage, ledit écart quadratique étant pondéré par le degré de certitude prédit par le décodeur d'état mental lors de la génération de ces données d'apprentissage, l'écart quadratique ainsi pondéré étant sommé sur l'ensemble des données d'apprentissage.

9. Méthode d'apprentissage d'une interface neuronale directe selon la revendication 3, **caractérisée en ce que** la prédiction effectuée par le modèle prédictif est basée sur une régression linéaire ou multilinéaire.

10. Méthode d'apprentissage d'une interface neuronale directe selon la revendication 9, **caractérisée en ce que**, si l'état mental prédit en un instant d'observation est un état d'erreur, les données d'apprentissage ne sont pas générées et que si cet état mental prédit est un état de satisfaction, les données d'apprentissage ne sont générées à partir du tenseur de données d'observation et du tenseur de données de commande à l'instant d'observation précédent, que si le degré de certitude de l'état mental prédit est supérieur à une première valeur de seuil prédéterminée ($Th^1_{mental\_state}$).

11. Méthode d'apprentissage d'une interface neuronale directe selon la revendication 9, **caractérisée en ce que**, quel que soit l'état prédit en un instant d'observation, les données d'apprentissage sont générées à partir du tenseur de données d'observation et du tenseur de données de commande à l'instant d'observation précédent, les données d'apprentissage étant alors associées avec le degré de certitude de la prédiction de l'état mental prédit ( $\left|\hat{y}^t_{mental\_state}\right|$ ).

12. Méthode d'apprentissage d'une interface neuronale directe selon la revendication 9, **caractérisée en ce que** la fonction de coût utilisée pour la mise à jour des paramètres du modèle prédictif dépend de l'écart quadratique entre le tenseur de données de commande prédit par le modèle prédictif et celui fourni par les données d'apprentissage, cette dépendance avec l'écart quadratique étant croissante lorsque l'état mental prédit lors de la génération des données d'apprentissage était un état de satisfaction et de manière décroissante lorsque cet état mental était un signal d'erreur, ledit écart quadratique étant pondéré par un facteur dépendant de manière croissante du degré de certitude de l'état mental prédit, associé aux données d'apprentissage.

**Patentansprüche**

1. Lernverfahren für eine direkte neuronale Schnittstelle, die dazu bestimmt ist, eine Mehrzahl von elektrophysiologischen Signalen zu empfangen, die eine neuronale Steuerung von einem Subjekt ausdrücken, während einer Mehrzahl von Beobachtungsfenstern, die Beobachtungszeitpunkten zugeordnet sind, wobei die elektrophysiologischen Signale in einem Vorverarbeitungsmodul (210, 310) vorverarbeitet werden, um zu jedem Beobachtungszeitpunkt einen Beobachtungsdatentensor zu bilden, wobei die direkte neuronale Schnittstelle ein Vorhersagemodell (220, 320) verwendet, um zu jedem Beobachtungszeitpunkt aus dem Beobachtungsdatentensor einen Steuerungsdatentensor abzuleiten, wobei die Steuerungsdaten dazu bestimmt sind, mindestens einen Effektor (230, 330) zu steuern, um eine Trajektorie zu verwirklichen, **dadurch gekennzeichnet, dass**:

   - Ein mentaler Zustand der Zufriedenheit oder des Irrtums des Subjekts (250, 350) zu jedem Beobachtungszeitpunkt mittels eines zuvor trainierten Decoders aus dem Beobachtungsdatentensor decodiert (250, 350) wird, wobei der mentale Zustand indikativ für die Übereinstimmung der Trajektorie mit der neuronalen Steuerung ist;
   - Lerndaten aus dem mentalen Zustand der Zufriedenheit oder des Irrtums, der zu einem bestimmten Beobachtungseitpunkt decodiert wurde, und aus dem Paar, das durch den Beobachtungsdatentensor und der Steuerungsdatensor zu einem früheren Zeitpunkt gebildet wurde, generiert (260, 360) werden;
   - die Parameter des Vorhersagemodells aktualisiert (220, 370) werden, indem eine Kostenfunktion für die im vorherigen Schritt generierten Lerndaten minimiert wird.

2. Lernverfahren für eine direkte neuronale Schnittstelle nach Anspruch 1, **dadurch gekennzeichnet, dass** der Decoder des mentalen Zustands in einer vorherigen Phase trainiert wird, indem dem Subjekt gleichzeitig eine Bewegungsanweisung und eine Trajektorie präsentiert werden, wobei der Beobachtungsdatentensor mit einem mentalen Zustand der Zufriedenheit gekennzeichnet wird, wenn die Trajektorie der Anweisung entspricht, und mit einem mentalen Zustand des Irrtums, wenn diese davon abweicht.

3. Lernverfahren für eine direkte neuronale Schnittstelle nach Anspruch 2, **dadurch gekennzeichnet, dass** der Decoder des mentalen Zustands zu jedem Beobachtungszeitpunkt eine Vorhersage des mentalen Zustands in Form eines Binärwerts ( $\hat{y}^t_{D,mental\_state}$ ) sowie eine Schätzung des Gewissheitsgrads dieser Vorhersage ( $\left|\hat{y}^t_{mental\_state}\right|$ ) bereitstellt.

4. Lernverfahren für eine direkte neuronale Schnittstelle nach Anspruch 3, **dadurch gekennzeichnet, dass** die vom Vorhersagemodell getroffene Vorhersage auf einer Klassifizierung basiert, wobei der Steuerungsdatentensor aus der vom Vorhersagemodell vorhergesagten wahrscheinlichsten Klasse erhalten wird.

5. Lernverfahren für eine direkte neuronale Schnittstelle nach Anspruch 4, **dadurch gekennzeichnet, dass**, wenn der zu einem Beobachtungszeitpunkt vorhergesagte mentale Zustand ein Zustand der Zufriedenheit ist, die Lerndaten nur aus dem Beobachtungsdatentensor und dem Steuerungsdatentensor zu dem früheren Zeitpunkt generiert werden, wenn der Gewissheitsgrad des vorhergesagten mentalen Zustands größer ist als ein erster vorbestimmter

Schwellenwert ($Th^1_{mental\_state}$).

6. Lernverfahren für eine direkte neuronale Schnittstelle nach Anspruch 4 oder 5, **dadurch gekennzeichnet, dass** der zu einem Beobachtungszeitpunkt vorhergesagte mentale Zustand ein Zustand des Irrtums ist, wobei die Lerndaten nur aus dem Beobachtungsdatentensor und dem Steuerungsdatentensor zu dem früheren Zeitpunkt generiert werden, wenn der Gewissheitsgrad des vorhergesagten mentalen Zustands größer ist als ein zweiter vorbestimmter

Schwellenwert ($Th^2_{mental\_state}$).

7. Lernverfahren für eine direkte neuronale Schnittstelle nach einem der Ansprüche 4 bis 6, **dadurch gekennzeichnet, dass**, wenn der zu einem Beobachtungszeitpunkt vorhergesagte mentale Zustand ein Fehlerzustand ist, die generierten Lerndaten den Beobachtungsdatentensor zum vorherigen Beobachtungszeitpunkt sowie einen Steuerungsdatentensor umfassen, der aus der zweitwahrscheinlichsten vorhergesagten Klasse erhalten wird, die von dem Vorhersagemodell zum vorherigen Beobachtungszeitpunkt vorhergesagt wurde.

8. Lernverfahren für eine direkte neuronale Schnittstelle nach einem der Ansprüche 4 bis 7, **dadurch gekennzeichnet, dass** die Kostenfunktion, die für die Aktualisierung der Parameter des Vorhersagemodells verwendet wird, die quadratische Abweichung zwischen dem vom Modell vorhergesagten Steuerungsdatentensor und dem von den Lerndaten bereitgestellten Tensor darstellt, wobei die quadratische Abweichung mit dem vorhergesagten Gewissheitsgrad durch den Decoder des mentalen Zustands während der Generierung dieser Lerndaten gewichtet wird, wobei die so gewichtete quadrierte Abweichung über die gesamten Lerndaten summiert wird.

9. Lernverfahren für eine direkte neuronale Schnittstelle nach Anspruch 3, **dadurch gekennzeichnet, dass** die vom Vorhersagemodell getroffene Vorhersage auf einer linearen oder multilinearen Regression basiert.

10. Lernverfahren für eine direkte neuronale Schnittstelle nach Anspruch 9, **dadurch gekennzeichnet, dass**, wenn der zu einem Beobachtungszeitpunkt vorhergesagte mentale Zustand ein Zustand des Irrtums ist, die Lerndaten nicht generiert werden, und dass, wenn dieser vorhergesagte mentale Zustand ein Zustand der Zufriedenheit ist, die Lerndaten nur aus dem Beobachtungsdatentensor und den Steuerungsdatentensor zu dem vorherigen Zeitpunkt generiert werden, wenn der Gewissheitsgrad des vorhergesagten mentalen Zustands größer ist als ein erster vorbestimmter Schwellenwert ($Th^1_{mental\ state}$).

11. Lernverfahren für eine direkte neuronale Schnittstelle nach Anspruch 9, **dadurch gekennzeichnet, dass** die Lerndaten unabhängig von dem zu einem Beobachtungszeitpunkt vorhergesagten Zustand aus dem Beobachtungsdatentensor und dem Steuerungsdatentensor zum vorherigen Beobachtungszeitpunkt generiert werden, wobei die Lerndaten dann dem Gewissheitsgrad der Vorhersage des vorhergesagten mentalen Zustands ($\left| \hat{y}^t_{mental\_state} \right|$) zugeordnet werden.

12. Lernverfahren für eine direkte neuronale Schnittstelle nach Anspruch 9, **dadurch gekennzeichnet, dass** die Kostenfunktion, die zur Aktualisierung der Parameter des Vorhersagemodells verwendet wird, von der quadratischen Abweichung zwischen dem vom Vorhersagemodell vorhergesagten Steuerungsdatentensor und dem von den Lerndaten bereitgestellten Tensor abhängen, wobei diese Abhängigkeit mit der quadratischen Abweichung zunimmt, wenn der während der Generierung der Lerndaten vorhergesagte mentale Zustand ein Zustand der Zufriedenheit war, und abnimmt, wenn dieser mentale Zustand ein Fehlersignal war, wobei die quadratische Abweichung mit einem Faktor gewichtet wird, der zunehmend vom Gewissheitsgrad des vorhergesagten mentalen Zustands abhängt, der den Lerndaten zugeordnet ist.

**Claims**

1. Method for training a brain computer interface intended to receive a plurality of electrophysiological signals expressing a neural command of a subject, during a plurality of observation windows associated with observation instants, said electrophysiological signals being preprocessed in a preprocessing module (210, 310) to form at each observation instant an observation data tensor, the brain computer interface using a predictive model (220, 320) to deduce at each observation instant a command data tensor from the observation data tensor, said command data being intended to control at least one effector (230, 330) to perform a trajectory, said training method being **characterized in that**:

   - at each observation instant, a satisfaction or error mental state of the subject (250, 350) is decoded (250, 350) from the observation data tensor by means of a decoder trained beforehand, said mental state being representative of the conformity of the trajectory with the neural command;
   - training data are generated (260, 360) from the satisfaction or error mental state, decoded at a given observation instant, and from the pair formed by the observation data tensor and the command data tensor at a preceding instant;
   - the parameters of the predictive model are updated (220, 370) by minimising a cost function on the training data generated at the preceding step.

2. Method for training a brain computer interface according to claim 1, **characterised in that** the mental state decoder is trained in a previous phase by presenting simultaneously to the subject a movement setpoint and a trajectory, the observation data tensor being labelled with a satisfaction mental state when the trajectory is in accordance with the setpoint and with an error mental state when it deviates therefrom.

3. Method for training a brain computer interface according to claim 2, **characterised in that** the mental state decoder provides at each observation instant a prediction of the mental state in the form of a binary value ( $\hat{y}^t_{D,mental\_state}$ ) as well as an estimation of the degree of certainty of this prediction ( $\left| \hat{y}^t_{mental\_state} \right|$ ).

4. Method for training a brain computer interface according to claim 3, **characterised in that** the prediction made by the predictive model is based on a classification, the command data tensor being obtained from the most probable class predicted by the predictive model.

5. Method for training a brain computer interface according to claim 4, **characterised in that** if the mental state predicted at an observation instant is a satisfaction state, the training data are generated from the observation data tensor and from the command data tensor at the preceding observation instant, only if the degree of certainty of the predicted mental state is greater than a first predetermined threshold value ( $Th^1_{mental\_state}$ ).

6. Method for training a brain computer interface according to claim 4 or 5, **characterised in that** if the mental state predicted at an observation instant is an error state, the training data are generated from the observation data tensor and from the command data tensor at the preceding observation instant, only if the degree of certainty of the predicted mental state is greater than a second predetermined threshold value ( $Th^2_{mental\_state}$ ).

7. Method for training a brain computer interface according to one of claims 4 to 6, **characterised in that** if the mental state predicted at an observation instant is an error state, the training data generated comprise the observation data tensor at the preceding observation instant as well as a command data tensor obtained from the second most probable class predicted by the predictive model at the preceding observation instant.

8. Method for training a brain computer interface according to one of claims 4 to 7, **characterised in that** the cost function used for updating the parameters of the predictive model expresses the square deviation between the command data tensor predicted by the model and that provided by the training data, said square deviation being weighted by the degree of certainty predicted by the mental state decoder during the generation of these training data, the square deviation thus weighted being summated over the whole training data set.

9. Method for training a brain computer interface according to claim 3, **characterised in that** the prediction made by the predictive model is based on a linear or multilinear regression.

10. Method for training a brain computer interface according to claim 9, **characterised in that**, if the mental state predicted at an observation instant is an error state, the training data are not generated and that if this predicted mental state is a satisfaction state, the training data are generated from the observation data tensor and from the command data tensor at the preceding observation instant, only if the degree of certainty of the predicted mental state is greater than the first predetermined threshold value ( $Th^1_{mental\_state}$ ).

11. Method for training a brain computer interface according to claim 9, **characterised in that**, regardless of the state predicted at an observation instant, the training data are generated from the observation data tensor and from the command data tensor at the preceding observation instant, the training data then being associated with the degree of certainty of the prediction of the predicted mental state ( $\left| \hat{y}^t_{mental\_state} \right|$ ).

12. Method for training a brain computer interface according to claim 9, **characterised in that** the cost function used for updating the parameters of the predictive model depends on the square deviation between the command data tensor predicted by the predictive model and that provided by the training data, this dependency with the square deviation being increasing when the mental state predicted during the generation of the training data was a satisfaction state and decreasing when this mental state was an error signal, said square deviation being weighted by a factor depending increasingly on the degree of certainty of the predicted mental state, associated with the training data.

FIG.1A

FIG.1B

EP 4 024 170 B1

FIG.2

EP 4 024 170 B1

FIG.3

## RÉFÉRENCES CITÉES DANS LA DESCRIPTION

**Documents brevets cités dans la description**

- FR 3061318 A

**Littérature non-brevet citée dans la description**

- **D. ROTERMUND et al.** Towards On-line Adaptation of Neuro-prostheses with Neuronal Evaluation Signals. *Biological Cybernetics - Advances In Computational Neuroscience,* Juin 2006, vol. 95 (3
- **T. GÜREL et al.** Unsupervised adaptation of brain-machine interface decoders. *Frontiers in Neuroscience,* Novembre 2012

- **A. KUMAR et al.** A Review of Error-Related Potential-Based Brain-Computer Interfaces for Motor Impaired People. *IEEE Access,* Septembre 2019, vol. 7
- **A. ELISEYEV et al.** Recursive exponentially weighted N-way Partial Least Squares régression with recursive validation of hyper-parameters in Brain-Computer Interface applications. *Scientific Reports,* Novembre 2017, vol. 7 (1), 16281